(19) Europäisches Patentamt
European Patent Office
Office européen des brevets

(11) Veröffentlichungsnummer: **0 138 772**
**A1**

(12) **EUROPÄISCHE PATENTANMELDUNG**

(21) Anmeldenummer: **84810501.1**

(22) Anmeldetag: **11.10.84**

(51) Int. Cl.⁴: **C 07 D 213/64,** A 01 N 47/40

(30) Priorität: **17.10.83 CH 5627/83**
**13.08.84 CH 3870/84**

(43) Veröffentlichungstag der Anmeldung: **24.04.85**
**Patentblatt 85/17**

(84) Benannte Vertragsstaaten: **CH DE FR GB IT LI**

(71) Anmelder: **CIBA-GEIGY AG, Postfach, CH-4002 Basel (CH)**

(72) Erfinder: **Böger, Manfred, Wilhelm Glock-Strasse 14, D-7858 Weil am Rhein 5 (DE)**
Erfinder: **Drabek, Jozef, Dr., Benkenstrasse 12, CH-4104 Oberwil (CH)**

(54) **Phenylcarboximidsäureester.**

(57) Neue substituierte N-[[[Pyridyloxyphenyl]-amino]-carbonyl]-phenylcarboximidsäureester der Formel

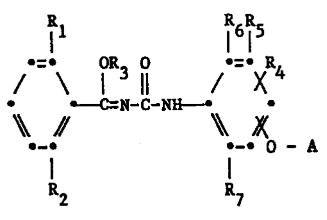

(I)

worin

$R_1$ Wasserstoff, Halogen, Methyl oder Methoxy;

$R_2$ Halogen, Methyl, Trifluormethyl oder Methoxy;

$R_3$ $C_1$-$C_5$-Alkyl, $C_1$-$C_3$-Halogenalkyl mit 1 bis 3 Halogenatomen, Allyl oder Propargyl;

$R_4$ und $R_5$ unabhängig voneinander Wasserstoff, Halogen, Methyl oder Trifluormethyl;

$R_6$ und $R_7$ Wasserstoff; und

A den Rest 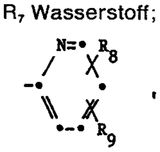 ,

wobei $R_8$ für Wasserstoff, Chlor, Trifluormethyl oder perhalogeniertes Äthyl und $R_9$ für Wasserstoff, Halogen, Methoxy oder Äthoxy stehen; bedeuten, Verfahren zur Herstellung dieser Verbindungen sowie diese enthaltende Mittel zur Bekämpfung von Insekten und Vertretern der Ordnung Akarina. Die neuen Verbindungen sind speziell gegen pflanzenschädigende Insekten wirksam.

EP 0 138 772 A1

CIBA-GEIGY AG
Basel (Schweiz)

5-14616/1+2

Phenylcarboximidsäureester

Die vorliegende Erfindung betrifft neue substituierte N-[[[Pyridyloxyphenyl]-amino]-carbonyl]-phenylcarboximidsäureester, Verfahren zu ihrer Herstellung und ihre Verwendung in der Schädlingsbekämpfung.

Die erfindungsgemässen Verbindungen haben die Formel I

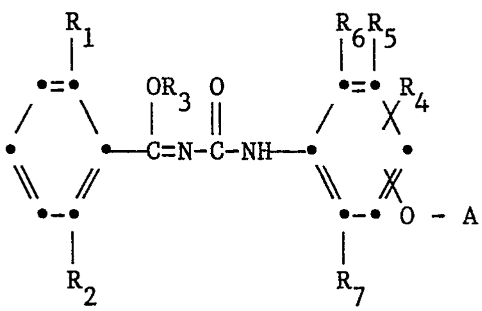

(I),

worin

$R_1$ Wasserstoff, Halogen, Methyl oder Methoxy;

$R_2$ Halogen, Methyl, Trifluormethyl oder Methoxy;

$R_3$ $C_1$-$C_5$-Alkyl, $C_1$-$C_3$-Halogenalkyl mit 1 bis 3 Halogenatomen, Allyl oder Propargyl;

$R_4$ und $R_5$ unabhängig voneinander Wasserstoff, Halogen, Methyl oder Trifluormethyl;

$R_6$ und $R_7$ Wasserstoff; und

A den Rest 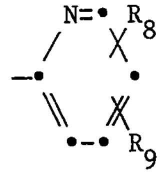 , wobei $R_8$ für Wasserstoff, Chlor,

Trifluormethyl oder perhalogeniertes Aethyl und $R_9$ für Wasserstoff, Halogen, Methoxy oder Aethoxy stehen;

bedeuten.

Bevorzugte Verbindungen der Formel I sind dadurch gekennzeichnet, dass
$R_1$ Wasserstoff, Halogen oder Methyl und
$R_2$ Halogen, Methyl oder Trifluormethyl
bedeuten; sowie auch dadurch,
dass A den Rest

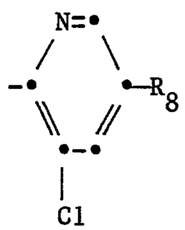

, wobei $R_8$ für Chlor, Trifluormethyl oder perhalogeniertes Aethyl steht,

bedeutet, oder dadurch, dass A den Rest

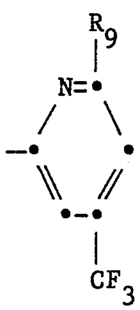

, wobei $R_9$ für Wasserstoff oder Chlor steht,

bedeutet.

Von besonderem Interesse wegen ihrer biologischen Wirkung sind solche Verbindungen der Formel I, worin $R_8$ Trifluormethyl oder eine der Gruppen $-CF_2CF_2Cl$, $-CF_2-CFCl_2$, $-CCl_2-CCl_3$, $-CF_2-CCl_3$ oder $-CF_2-CF_3$ bedeutet; solche, worin $R_9$ Chlor bedeutet; solche, worin die Gruppe -O-A in 4-Stellung am Phenylring steht; und solche, worin die Gruppe -O-A- in 5-Stellung am Phenylring steht.

Speziell bevorzugt sind Verbindungen der Formel I, worin
$R_1$ Wasserstoff, Fluor, Chlor oder Methyl;
$R_2$ Fluor, Chlor oder Trifluormethyl;
$R_3$ Methyl, Aethyl, n-Propyl, i-Propyl, $-CH_2CF_3$ oder $-CH_2CCl_3$;
$R_4$ Wasserstoff, Fluor, Chlor, Brom oder Methyl;
$R_5$ Wasserstoff, Fluor oder Chlor; und
$R_6$ und $R_7$ Wasserstoff
bedeuten; oder worin
$R_1$ Wasserstoff, Fluor oder Chlor;
$R_2$ Fluor;
$R_3$ Methyl, Aethyl oder n-Propyl; und
$R_4$ und $R_5$ unabhängig voneinander Wasserstoff, Fluor oder Chlor
bedeuten; oder worin
$R_3$ Methyl oder Aethyl und
$R_4$ und $R_5$ unabhängig voneinander Wasserstoff oder Chlor
bedeuten.

Von Interesse sind weiterhin Verbindungen der Formel I, die dadurch gekennzeichnet sind, dass
$R_1$ Wasserstoff oder Brom und
$R_2$ Brom
bedeuten; oder dadurch, dass
$R_4$ Wasserstoff oder Methyl und
$R_5$ Methyl
bedeuten; oder dadurch, dass
$R_3$ $C_1$-$C_3$-Halogenalkyl mit 1 bis 3 Halogenatomen, Allyl oder Propargyl
bedeutet.

Unter dem Begriff Alkyl sind geradkettige und verzweigte Alkylreste und je nach Zahl der angegebenen Kohlenstoffatome beispielsweise folgende Gruppen zu verstehen: Methyl, Aethyl, Propyl, Butyl und Pentyl, sowie ihre Isomeren, wie z.B. Isopropyl, Isobutyl, tert.-Butyl, Isopentyl usw.

Die erfindungsgemässen Verbindungen der Formel I können in zwei unterschiedlichen geometrischen Isomeren vorliegen, und zwar in Abhängigkeit von der räumlichen Anordnung der Substitutions-Gruppierungen an der -C=N-Bindung. Vereinbarungsgemäss werden solche Isomeren als E-Form und Z-Form bezeichnet. Für die Verbindungen der Formel I stellen sich die beiden isomeren Formen wie folgt dar:

Z-Form:

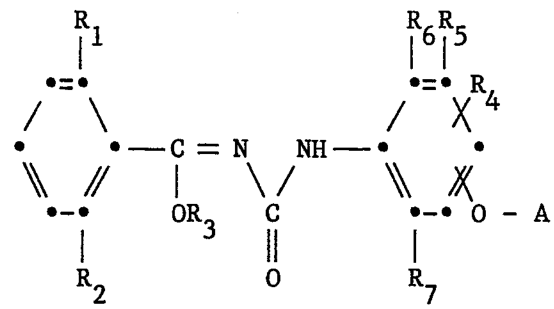

E-Form:

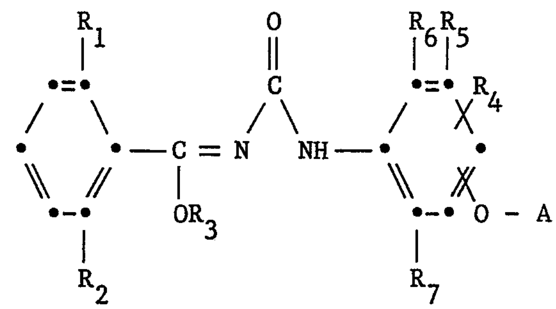

Die Verbindungen der Formel I können je nach ihrer Raumstruktur als reine Z-Form, reine E-Form oder als Gemische beider Formen vorliegen.

Die Verbindungen der Formel I können analog an sich bekannten Verfahren (vgl.US-Patentschrift No. 4.357.347 und europäische Patentschrift No. 0.005.944) hergestellt werden.

So kann man z.B. eine Verbindung der Formel I erhalten, indem man

a) eine Verbindung der Formel II

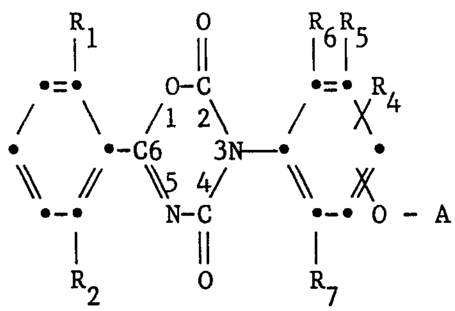

(II)

mit einer Verbindung der Formel III

$R_3$—OH (III)

oder

b) eine Verbindung der Formel IV

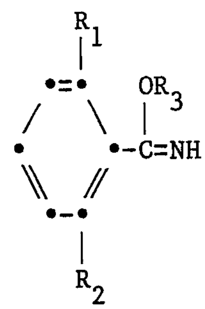

(IV)

mit einer Verbindung der Formel V

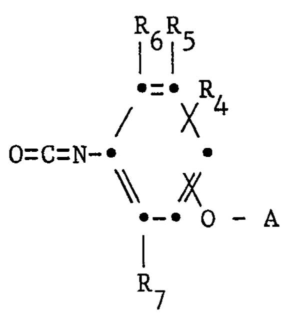

(V)

oder mit Phosgen und einer Verbindung der Formel VI

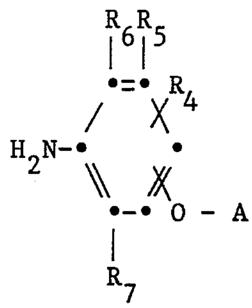

(VI)

umsetzt, wobei in den Formeln II bis VI die Reste $R_1$ bis $R_7$ und A die vorstehend unter Formel I angegebenen Bedeutungen haben.

Das vorerwähnte Verfahren a) wird im allgemeinen unter Normaldruck und vorzugsweise durch direktes Einwirkenlassen der Reaktionspartner durchgeführt, wobei die Temperatur im Bereich von 20° bis 120°C, vorzugsweise von 30° bis 90°C, liegt. Die Umsetzung kann auch in Gegenwart von gegenüber den Reaktionsteilnehmern inerten Lösungsmitteln ausgeführt werden. Vorteilhafterweise verwendet man einen Ueberschuss des Alkohols der Formel III als Lösungsmittel. Bei Verfahren b) wird gewöhnlich unter Normaldruck, in Gegenwart eines geeigneten inerten Lösungsmittels und bei einer Temperatur von 0° bis 110°C, vorzugsweise von 20° bis 70°C, gearbeitet. Gewünschtenfalls kann zu dem Reaktionsgemisch eine katalytische Menge einer tertiären organischen Base, wie Triäthylamin, hinzugefügt werden. Verfahren b) kann auch variiert werden durch Phosgenisierung des Phenylcarboximidsäureesters der Formel IV bei einer Temperatur zwischen -20 und 60°C, und anschliessende Umsetzung des erhaltenen Chlorcarbonyl-phenylcarboximidsäureesters der Formel VII

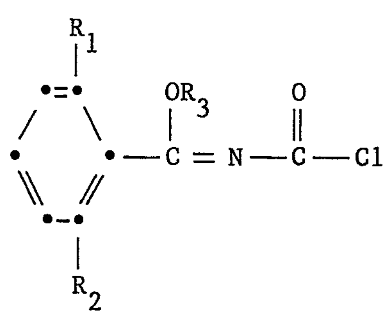

(VII)

mit einem entsprechend substituierten Pyridyloxyanilin der Formel VI in Gegenwart eines Säureacceptors, z.B. einer anorganischen oder organischen Base, wie Triäthylamin, Pyridin, Cholin usw. Als Lösungsmittel für die obigen Umsetzungen eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; Ester, wie Aethylacetat; aliphatische aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; sowie Ketone, z.B. Methyläthylketon, Methylisopropylketon und Methylisobutylketon.

Die für die erfindungsgemässen Herstellungsverfahren verwendeten Ausgangsprodukte der Formeln II bis VI sind bekannt oder können analog bekannten Arbeitsweisen erhalten werden. So kann man zu den 3,6-Diphenyl-1,3,5-oxadiazin-2,4-dion-Derivaten der Formel II gelangen (vgl. deutsche Offenlegungsschriften Nr. 2732115 und 2905687), indem man ein Pyridyloxyphenylisocyanat der Formel V mit einem Benzoylisocyanat der Formel VIIa

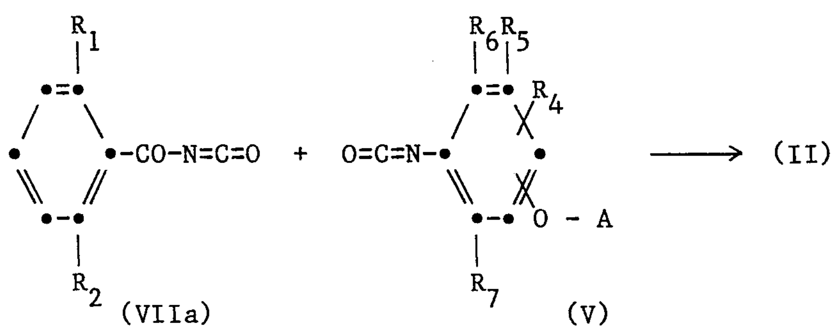

(VIIa) (V) (II)

oder mit einem Halogencarbonylbenzamid der Formel VIII umsetzt

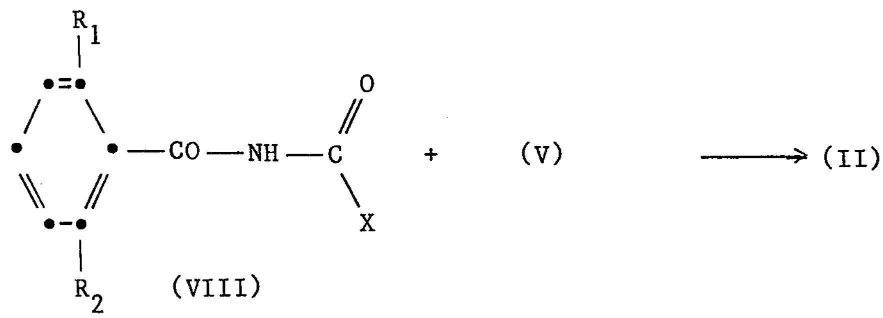

(VIII) (V) (II)

Die erwähnten Isocyanate der Formel V können aus den entsprechend substituierten Pyridyloxyanilinen der Formel VI durch übliche Umsetzung mit Phosgen erhalten werden. Zu den Pyridyloxyanilinen der Formel VI kann man z.B. gelangen, indem man ein umsetzungsfähiges Pyridin der nachstehenden Formel IX mit einem entsprechend substituierten Aminophenol in Gegenwart einer basischen Substanz umsetzt (vgl. die europäische Patentanmeldung Nr. 0 077 759 und die deutschen Offenlegungsschriften Nr. 3 240 975 und 3 241 138). Man kann die Aniline der Formel VI auch durch Umsetzung entsprechend substituierter Nitrophenole der Formel X mit einem Pyridin der Formel IX und anschliessende Reduktion der Nitrogruppe in der entstehenden Verbindung der Formel XI nach einem der gebräuchlichen Verfahren erhalten [vgl. z.B. Rec. 21, 271 (1902); J. Am. Soc. 68, 1604 (1964); J. Org. Chem. 11, 378 (1946); Rec. 79, 994 (1970)]:

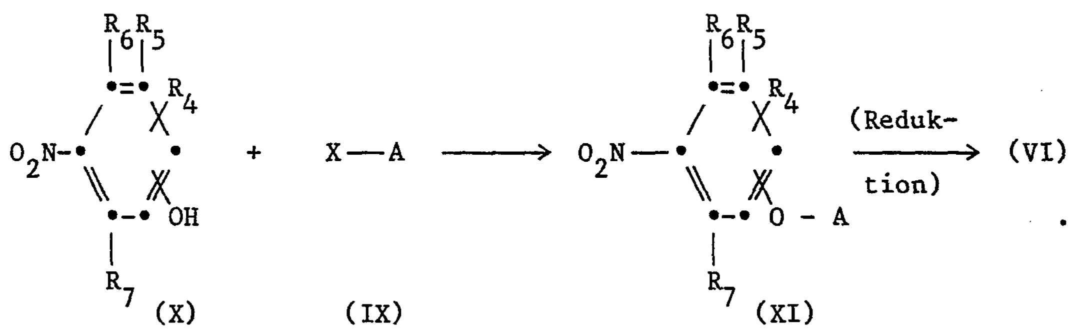


(X) (IX) (XI)

In den obigen Formeln VII bis XI entsprechen $R_1$ bis $R_7$ sowie A den vorstehend unter Formel I angegebenen Definitionen und X bedeutet Halogen, vorzugsweise Chlor. Phenylcarboximidsäureester der Formel IV sind durch Esterbildung an den entsprechenden Benzamiden in an sich bekannter Weise zugänglich (vgl. z.B. US-Patentschrift Nr. 4,357,347).

Aus der US-Patentschrift Nr. 4,357,347 und der europäischen Patentschrift Nr. 0005944 sind bereits unterschiedlich substituierte Ester der N-[[[Phenoxyphenyl]-amino]-carbonyl]-phenylcarboximidsäure mit insektizider Wirkung bekannt. Demgegenüber wurde gefunden, dass die erfindungsgemässen Verbindungen der Formel I, die als ein wesentliches Strukturelement eine Pyridyloxyphenyl-Gruppierung enthalten, überraschenderweise ausgezeichnete Wirkung als Pestizide,

insbesondere als Insektizide, aufweisen. Die erfindungsgemässen substituierten N-[[[Pyridyloxyphenyl]-amino]-carbonyl]-phenylcarboximidsäureester sind vor allem wertvoll als Insektizide im Pflanzenschutz. Ein besonderer Vorteil der erfindungsgemässen Verbindungen der Formel I ergibt sich aus ihrer sehr geringen Warmblütertoxizität bei guter Pflanzenverträglichkeit.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien: Ixodidae, Argasidae, Tetranychidae und Dermanyssidae.

Neben ihrer Wirkung gegenüber Fliegen, wie z.B. Musca domestica, und Mückenlarven können Verbindungen der Formel I auch zur Bekämpfung von pflanzenschädigenden Frassinsekten, speziell ihrer larvalen Stadien, in Zier- und Nutzpflanzungen, insbesondere in Baumwollkulturen (z.B. Spodoptera littoralis und Heliothis virescens), sowie in Obst- und Gemüsekulturen (z.B. Laspeyresia pomonella, Leptinotarsa decemlineata und Epilachna varivestis) eingesetzt werden. Die Verbindungen der Formel I zeichnen sich auch durch ovizide und larvizide Wirkung gegen Insekten, insbesondere Larven von fressenden Schadinsekten, aus. Werden Verbindungen der Formel I von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I können ferner zur Bekämpfung von Ektoparasiten, wie Lucilia sericata, an Haus- und Nutztieren eingesetzt werden, z.B. durch Tier-, Stall- und Weidebehandlung.

Die Verbindungen der Formel I eignen sich ferner zur Bekämpfung der folgenden, Obst- und Gemüsekulturen befallenden Milbenspezies: Tetranychus urticae, Tetranychus cinnabarinus, Panonychus ulmi,

Bryobia rubrioculus, Panonychus citri, Eriophyes piri, Eriophyes ribis, Eriophyes vitis, Tarsonemus pallidus, Phyllocoptes vitis und Phyllocoptruta oleivora.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht: organische Phosphorverbindungen, Nitrophenole und Derivate, Formamidine, Harnstoffe, Pyrethroide, Carbamate, chlorierte Kohlenwasserstoffe und Bacillus thuringiensis-Präparate.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methlendioxyphenoxy)-3,6,9-trioxaundecan oder S,S,S-Tributylphosphorotrithioate.

Die gute pestizide Wirkung der erfindungsgemässen Verbindungen der Formel I entspricht einer Abtötungsrate (Mortalität) von mindestens 50-60 % der erwähnten Schädlinge, insbesondere Schadinsekten.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthylähter, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden.

Als gekörnte, adsorptive Granulatträger kommen poröse Typen, wie z.B. Bimsstein, Zielgelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur, wie insbesondere Dolomit oder zerkleinerte Pflanzenrückstände verwendet, werden.

Als oberflächenaktive Verbindungen kommen je nach der Art des zu formulierenden Wirkstoffes der Formel I oder der Kombinationen dieser Wirkstoffe und anderen Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind als Tenside auch die Fettsäuremethyl-taurinsalze sowie modifizierte und nicht-modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsäure der Dodecylbenzolsulfonsäure, der Dibutylnaphthalinsulfonsäure oder einer Naphthalinsulfonsäure, der Dibutylnaphthalinsulfonsäure oder

eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalklreste aufweisen. Die Salze liegen vorzugweise als Halogenide, Methylsulfate oder Aethlsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents und Emulsifiers Annual", MC Publishing Corp., Ridgewood, New Jersey, 1979. Dr. Helmut Stache, "Tensid-Taschenbuch", C. Hauser Verlag, München/Wien (1981).

Die pestiziden Zubereitungen enthalten in der Regel 0,1 bis 99 %, insbesondere 0,1 bis 95 %, Wirkstoff der Formel I oder Kombinationen dieser Wirkstoffe mit anderen Insektiziden oder Akariziden, 1 bis 99,9 % eines festen oder flüssigen Zusatzstoffes und 0 bis 25 %, insbesondere, 0,1 bis 20 %, eines Tensides. Während als Handelsware eher konzentrierte Mittel, bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

<u>Formulierungsbeispiele für Wirkstoffe der Formel I resp. Kombinationen dieser Wirkstoffe mit anderen Insektiziden oder Akariziden (% = Gewichtsprozent)</u>

| 1. <u>Spritzpulver</u> | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - % |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2. Emulsions-Konzentrat

| | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliesend im Luftstrom getrocknet.

5. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1: Herstellung des 2-Chlor-N-[[[3,5-dichlor-4-(3-chlor-5-trifluormethyl-2-pyridyloxy)-phenyl]-amino]-carbonyl]-phenyl-carboximidsäuremethylesters:

Unter Ausschluss von Feuchtigkeit werden 4,0 g 3-[3,5-Dichlor-4-(3-chlor-5-trifluormethyl-2-pyridyloxy)-phenyl]-6-( 2-chlorphenyl)-3,4-dihydro-2H-1,3,5-oxadiazin-2,4-dion in 70 ml abs. Methylalkohol während 8 Stunden bei 60°C verrührt. Die erhaltene Lösung wird eingedampft und der Rückstand mit Dichlormethan an Kieselgel

chromatographiert. Der Rückstand aus den eingedampften Chromatographie-Eluaten wird mit Hexan verrieben und abgesaugt. Man erhält die Titelverbindung (Verbindung Nr. 1) der Formel

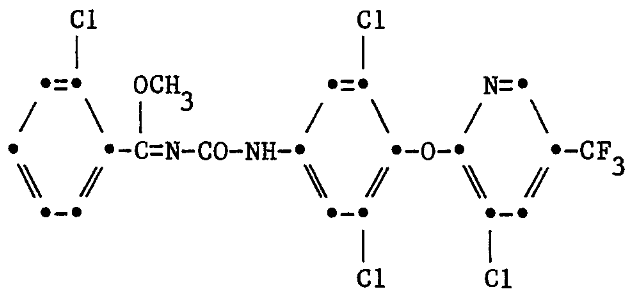

als weisses Pulver mit einem Schmelzpunkt von 147-149°C.

Analog den vorstehend beschriebenen Arbeitsweisen werden die folgenden Verbindungen der Formel I hergestellt:

| Verbindung Nr. | | physikalische Daten |
|---|---|---|
| 2 | 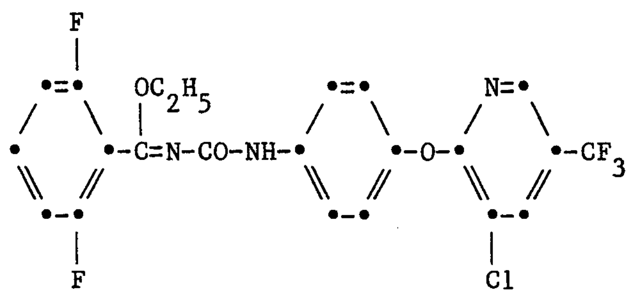 | Smp. 134-136°C |
| 3 | 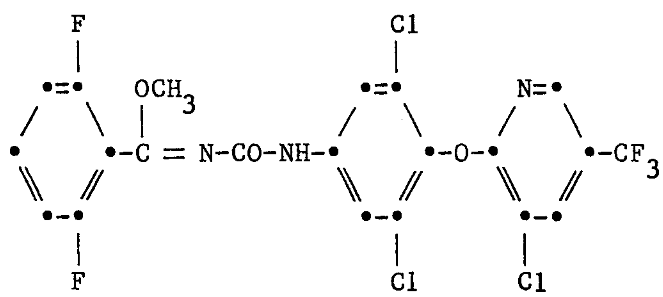 | Smp. 186-187°C |

0138772

| Verbindung Nr. | | physikalische Daten |
|---|---|---|
| 4 | 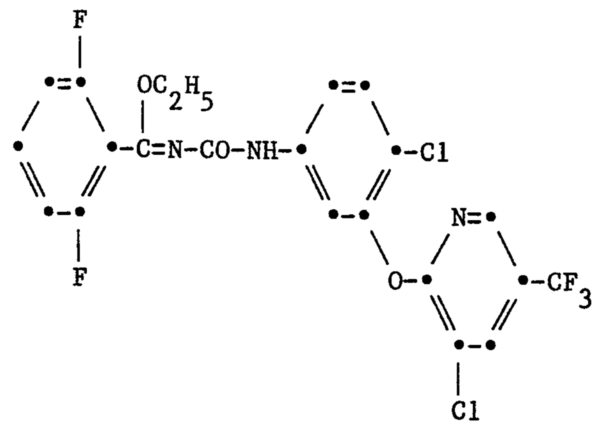 | Smp. 169-170°C |
| 5 | 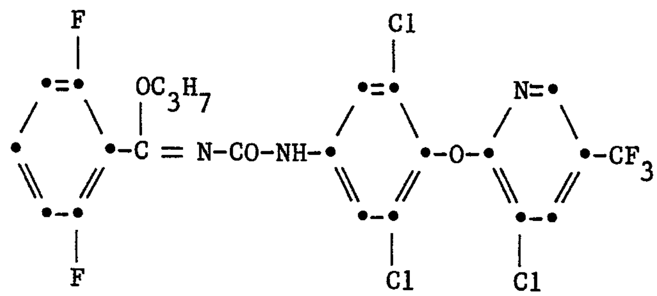 | amorphe Masse |
| 6 | 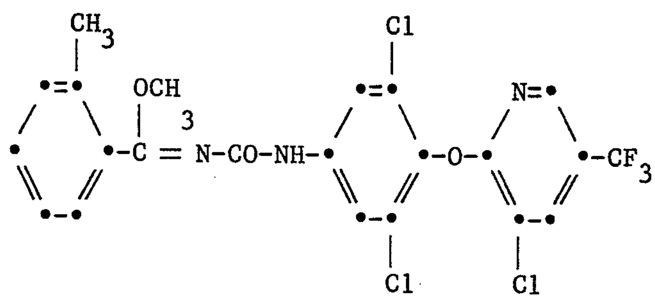 | Smp. 172-174°C |
| 7 | 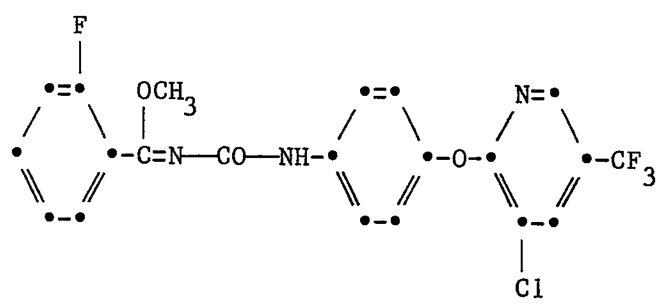 | Smp. 155-157°C |

| Verbindung Nr. | | physikalische Daten |
|---|---|---|
| 8 | 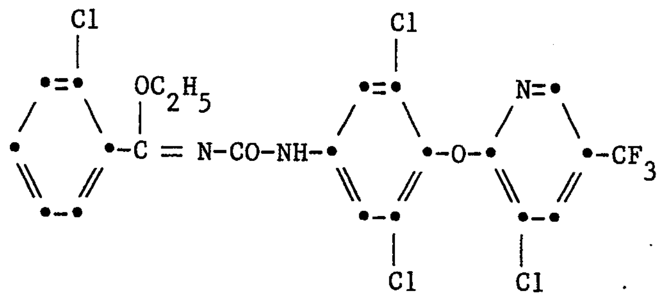 | Smp. 138-141°C |
| 9 | 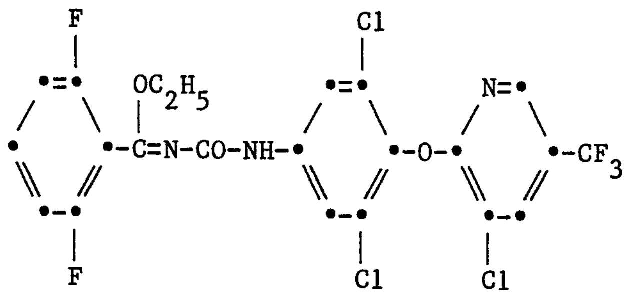 | Smp. 162-164°C |
| 10 | 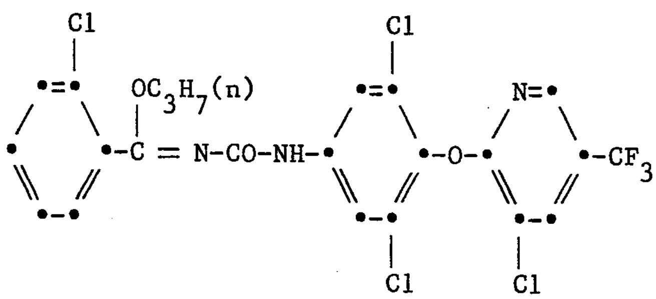 | amorphe Masse |
| 11 | 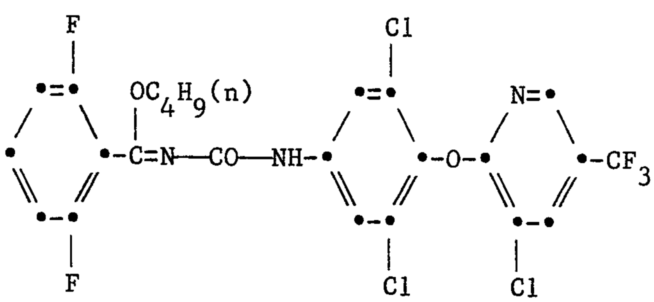 | amorphe Masse |

| Verbindung Nr. | | physikalische Daten |
|---|---|---|
| 12 | 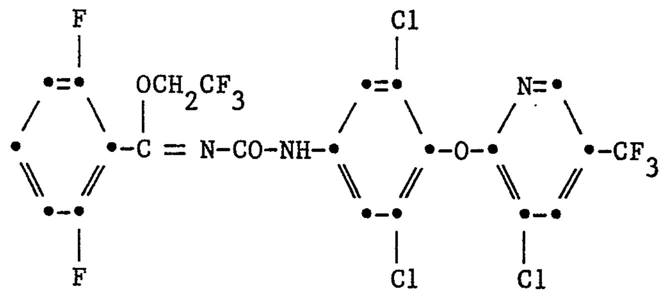 | Smp. 169-170°C |
| 13 | 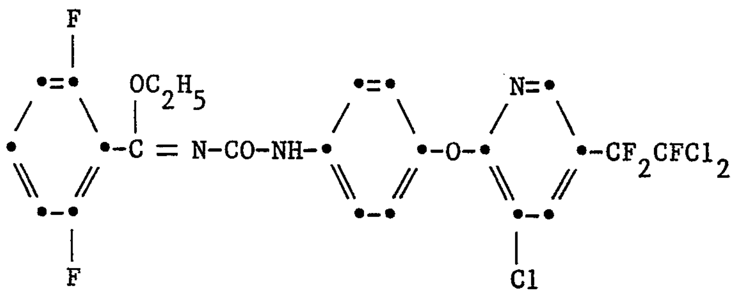 | Smp.162-164°C |
| 14 | 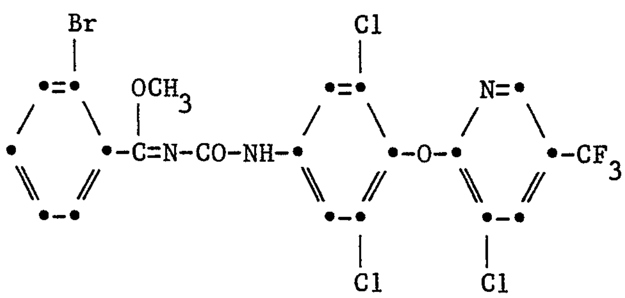 | Smp. 154-156°C |
| 15 | 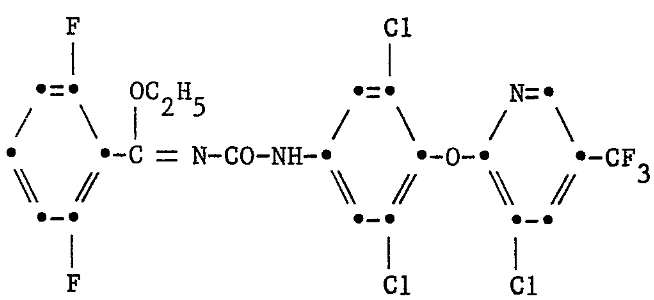 | Smp. 157-159°C |

| Verbindung Nr. | | physikalische Daten |
|---|---|---|
| 16 | 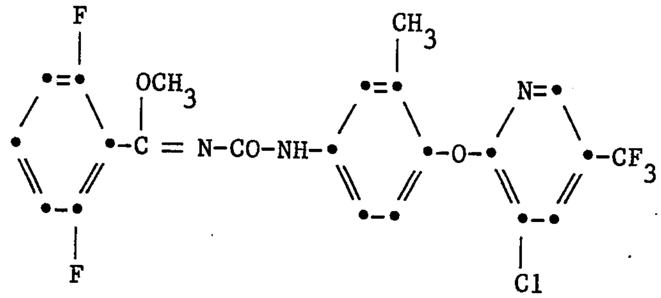 | Smp. 142-144°C |
| 17 | 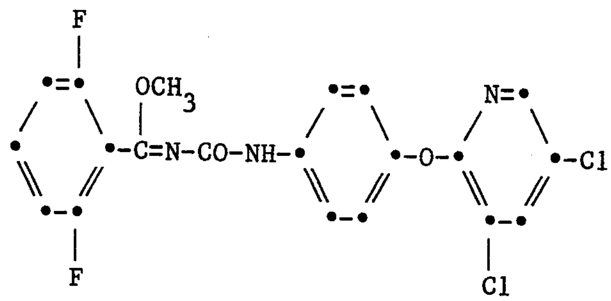 | Smp. 173-174°C |
| 18 | 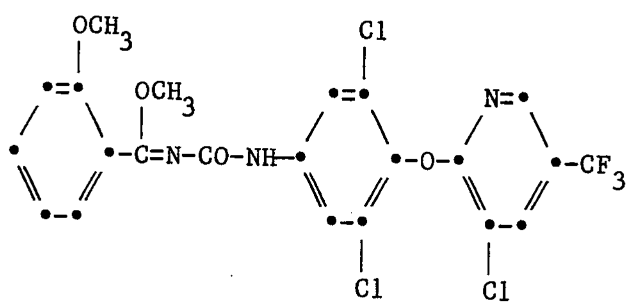 | Smp. 184-186°C |
| 19 | 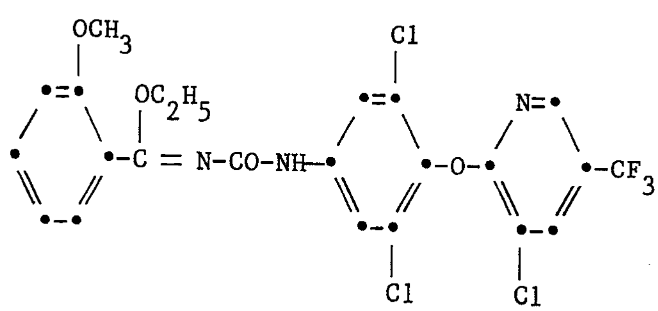 | Smp. 151-153°C |

| Verbindung Nr. | | physikalische Daten |
|---|---|---|
| 20 | 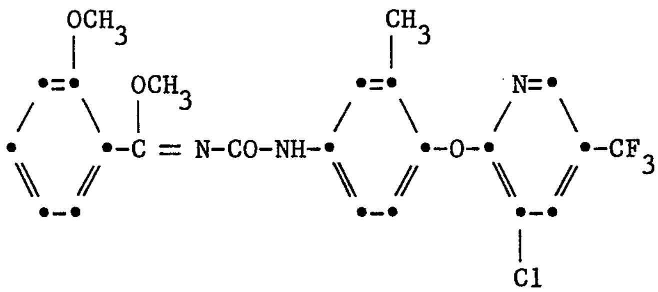 | Smp. 165-167°C |

In entsprechender Weise lassen sich die folgenden Verbindungen der Formel I herstellen:

| Verbindung Nr. | |
|---|---|
| 21 | 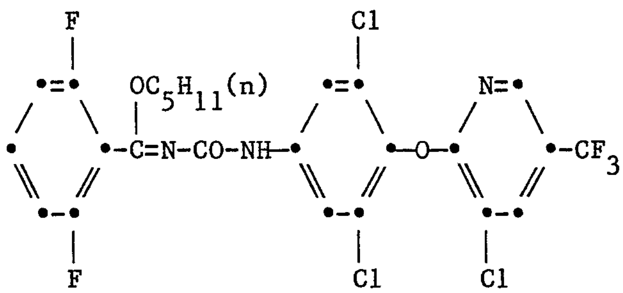 |
| 22 | 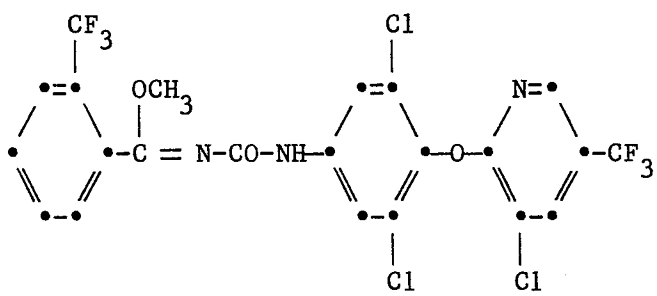 |
| 23 | 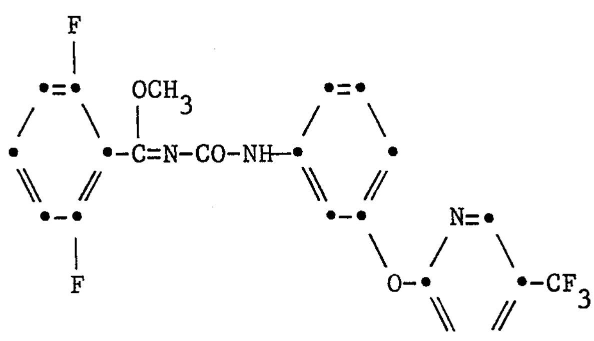 |

Verbindung
Nr.

24
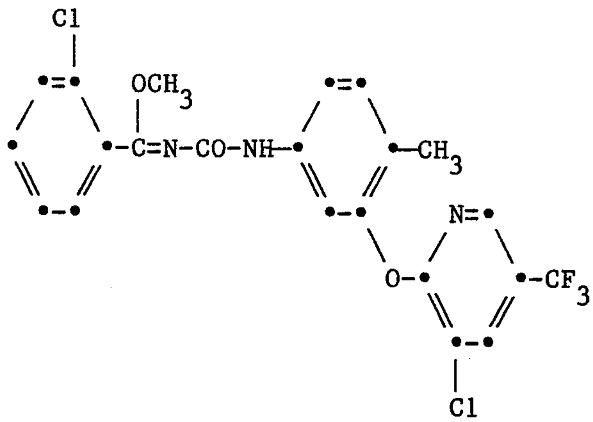

25
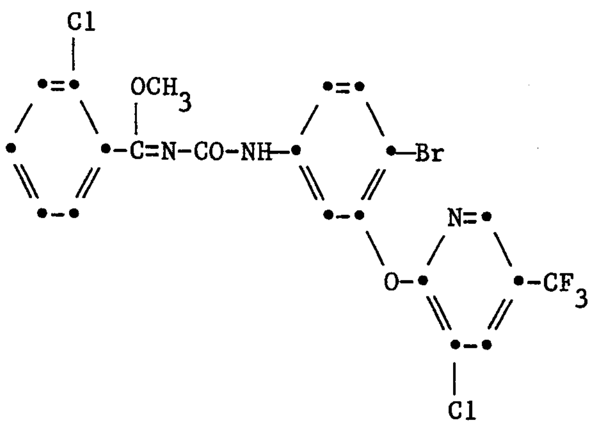

26
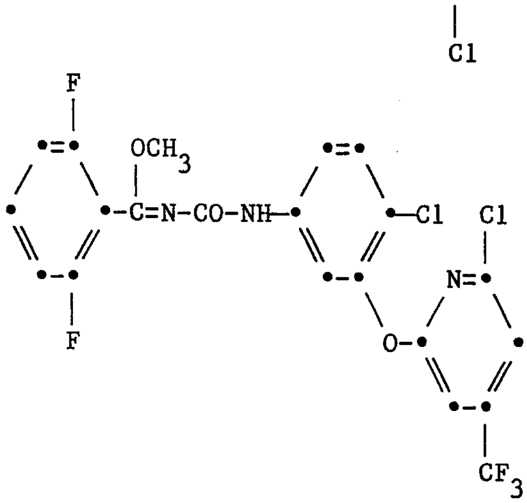

Verbindung
Nr.

27 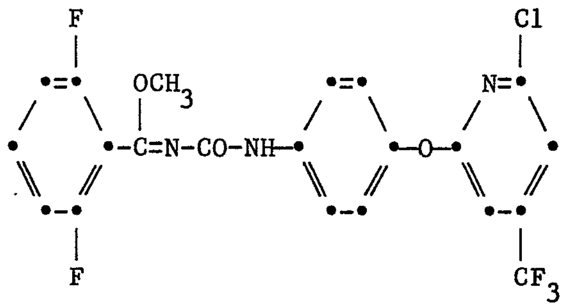

28 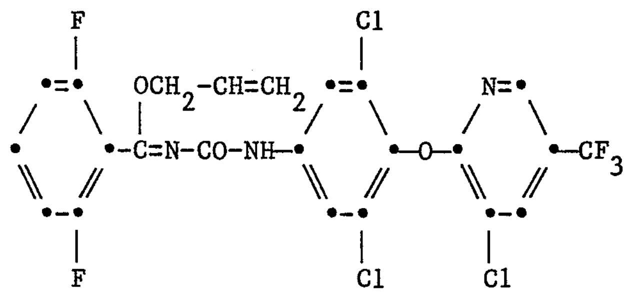

29 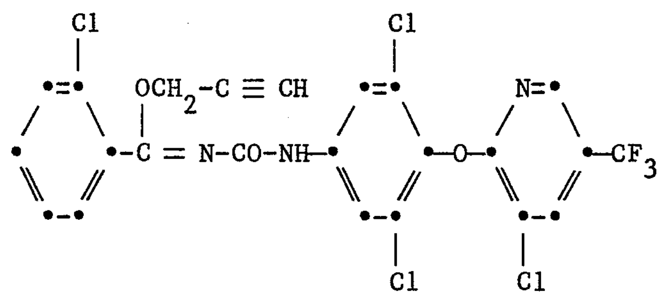

30 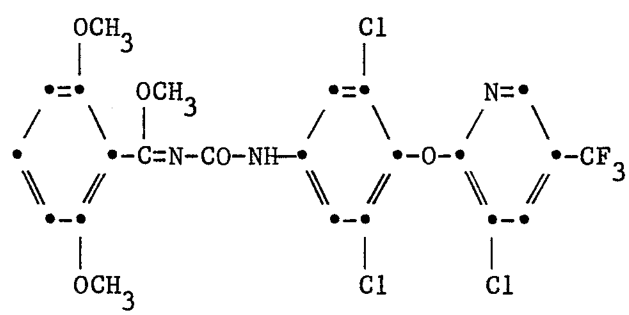

Beispiel 2: Wirkung gegen Musca domestica: Je 50 g frisch zubereitetes Nährsubstrat für Maden wird in Becher eingewogen. Von einer 1 Gew.%-igen acetonischen Lösung des betreffenden Wirkstoffes wird eine bestimmte Menge auf das in den Bechern befindliche Nährsubstrat

pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 zeigen gute Wirkung im obigen Test.

<u>Beispiel 3</u>: <u>Wirkung gegen Lucilia sericata</u>: Zu 9 ml eines Zuchtmediums werden bei 50°C 1 ml einer 0,5 Gew.-% Aktivsubstanz enthaltenden wässrigen Zubereitung gegeben. Nun werden ca. 30 frisch geschlüpfte Lucilia sericata-Larven zum Zuchtmedium gegeben, und nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

<u>Beispiel 4</u>: <u>Wirkung gegen Aëdes aegypti</u>: Auf die Oberfläche von 150 ml Wasser, das sich in einem Behälter befindet, wird so viel einer 0,1 Gew.-%igen acetonischen Lösung des Wirkstoffes pipettiert, dass eine Konzentration von 400 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formel I gemäss Beispiel 1 zeigen in diesem Test gute Wirkung gegen Aëdes aegypti.

Beispiel 5: Insektizide Frassgift-Wirkung: Ca. 25 cm hohe eingetopfte Baumwollpflanzen werden mit wässrigen Wirkstoffemulsionen besprüht, die den Wirkstoff in Konzentrationen von 400, 200, 100, 50, 12.5 und 3 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24°C und 60 % relativer Luftfeuchtigkeit durchgeführt. Nach 120 Stunden wird die %-Mortalität der Test-Insekten bestimmt.

Beispiel 6: Wirkung gegen Epilachna varivestis: Etwa 12-20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28°C und 60 % relativer Luftfeuchtigkeit durchgeführt.

Nach 2 und 3 Tagen wird die %-Mortalität bestimmt. Zur Auswertung hinsichtlich allfälligem Frass-Schaden (Antifeeding-Effekt), Entwicklungs- und Häutungsstörungen werden die Versuchstiere während weiterer 3 Tage beobachtet.

Verbindungen der Formel I gemäss Beispiel 1 zeigen gute Wirkung im obigen Test.

Beispiel 7: Ovizide Wirkung auf Heliothis virescens: Entsprechende Mengenanteile einer benetzbaren pulverförmigen Formulierung, enthaltend 25 Gew.% des zu prüfenden Wirkstoffes, werden mit jeweils soviel Wasser vermischt, dass sich wässrige Emulsionen von Wirkstoffkonzentrationen von 400 ppm ergeben.

In diese wirkstoffhaltigen Emulsionen werden eintägige Eigelege von Heliothis auf Cellophan während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt.

Verbindungen der Formel I gemäss Beispiel 1 zeigen gute Wirkung in obigem Test.

Beispiel 8: Wirkung auf Laspeyresia pomonella (Eier): Abgelegte Eier von Laspeyresia pomonella, die nicht älter als 24 Stunden sind, werden auf Filterpapier für 1 Minute in eine acetonisch-wässrige Lösung, enthaltend 800 ppm des zu prüfenden Wirkstoffes, eingetaucht. Nach dem Antrocknen der Lösung werden die Eier in Petrischalen ausgelegt und bei einer Temperatur von 28°C belassen. Nach 6 Tagen wird der prozentuale Schlupf aus den behandelten Eiern bewertet und die %-Mortalität bestimmt.

Verbindungen der Formel I gemäss Beispiel 1 zeigen gute Wirkung in obigem Test.

Beispiel 9: Reproduktions-Beeinflussung von Anthonomus grandis: Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 1,0 Gew.% des zu prüfenden Wirkstoffes, eingetaucht.

Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraumes von etwa 4 Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I gemäss Beispiel 1 zeigen eine gute reproduktionsreduzierende Wirkung im obigen Test.

<u>Beispiel 10</u>: <u>Akarizide Wirkung</u>: Phaseolus vulgaris Pflanzen werden 12 Stunden vor dem Test auf akarizide Wirkung mit einem infestierten Blattstück aus einer Massenzucht von Tetranychus urticae belegt. Die übergelaufenen beweglichen Stadien werden aus einem Chromatographiezerstäuber mit emulgierten Testpräparaten enthaltend 800 ppm Wirkstoff derart besprüht, dass kein Ablaufen der Spritzbrühe eintritt. Nach zwei und 7 Tagen werden Larven, Adulte und Eier unter dem Binokular auf lebende und tote Individuen ausgwertet; das Ergebnis wird in Prozenten ausgedrückt.

Während der "Haltezeit" stehen die behandelten Pflanzen in Gewächshauskabinen bei 25°C.

Verbindungen der Formel I gemäss Beispiel 1 zeigen im obigen Test gute Wirkung.

Beispiel 11: Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100, 50 bzw. 12.5 ppm des zu prüfenden Wirkstoffes besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung im Vergleich zu unbehandelten Kontrollansätzen.

Biologische Ergebnisse:

Die nachstehende Tabelle zeigt Ergebnisse biologischer Prüfungen erfindungsgemässer Verbindungen auf der Basis obiger biologischer Beispiele. Die Auswertung der Versuche erfolgt anhand der erhaltenen %-Mortalität unter Verwendung des folgenden Bewertungs-Index:

A: 80-100 % Mortalität bei einer Konzentration von 3,0 ppm der geprüften Verbindung;

B: 80-100 % Mortalität bei einer Konzentration von 12,5 ppm der geprüften Verbindung;

C: 80-100 % Mortalität bei einer Konzentration von 50 ppm der geprüften Verbindung;

D: 80-100 % Mortalität bei einer Konzentration von 100 ppm der geprüften Verbindung;

E: 80-100 % Mortalität bei einer Konzentration von 200 ppm der geprüften Verbindung;

F: 80-100 % Mortalität bei einer Konzentration von 400 ppm der geprüften Verbindung;

-: nicht geprüft.

| Verb. Nr. | pestizide Wirksamkeit | | |
|---|---|---|---|
| | Spodoptera-Larven (Beispiel 5) | Heliothis-Larven (Beispiel 5) | Anthonomus (Beispiel 11) |
| 1 | B | C | B |
| 2 | B | F | C |
| 3 | D | F | B |
| 4 | E | F | - |
| 5 | C | E | D |
| 6 | F | - | - |
| 7 | B | F | C |
| 8 | C | D | C |
| 9 | E | - | C |
| 10 | C | D | - |
| 11 | E | - | - |
| 13 | C | D | - |
| 14 | A | E | - |
| 15 | D | - | - |
| 16 | B | C | B |
| 17 | F | - | - |

Patentansprüche

1. Verbindung der Formel I

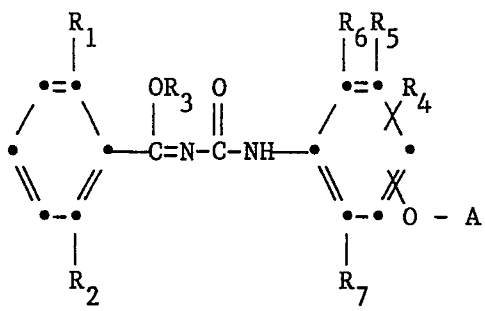

(I),

worin

$R_1$ Wasserstoff, Halogen, Methyl oder Methoxy;

$R_2$ Halogen, Methyl, Trifluormethyl oder Methoxy;

$R_3$ $C_1$-$C_5$-Alkyl, $C_1$-$C_3$-Halogenalkyl mit 1 bis 3 Halogenatomen, Allyl oder Propargyl;

$R_4$ und $R_5$ unabhängig voneinander Wasserstoff, Halogen, Methyl oder Trifluormethyl;

$R_6$ und $R_7$ Wasserstoff; und

A den Rest 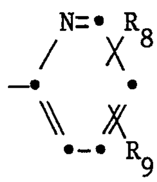 , wobei $R_8$ für Wasserstoff, Chlor, Trifluormethyl oder perhalogeniertes Aethyl und $R_9$ für Wasserstoff, Halogen, Methoxy oder Aethoxy stehen,

bedeuten.

2. Verbindung der Formel I nach Anspruch 1, dadurch gekennzeichnet, dass

$R_1$ Wasserstoff, Halogen oder Methyl und

$R_2$ Halogen, Methyl oder Trifluormethyl

bedeuten.

3. Verbindung der Formel I nach Anspruch 1 oder 2, dadurch gekennzeichnet, dass A den Rest

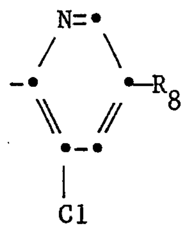

, wobei $R_8$ für Chlor, Trifluormethyl oder perhalogeniertes Aethyl steht,

bedeutet.

4. Verbindung der Formel I nach Anspruch 1 oder 2, dadurch gekennzeichnet, dass A den Rest

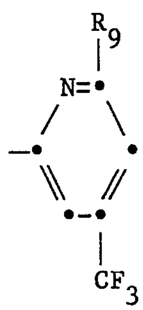

, wobei $R_9$ für Wasserstoff oder Chlor steht,

bedeutet.

5. Verbindung der Formel I nach Anspruch 3, dadurch gekennzeichnet, dass $R_8$ Trifluormethyl oder eine der Gruppen $-CF_2CF_2Cl$, $-CF_2-CFCl_2$, $-CCl_2-CCl_3$, $-CF_2-CCl_3$ oder $-CF_2-CF_3$ bedeutet.

6. Verbindung der Formel I nach Anspruch 4, dadurch gekennzeichnet, dass $R_9$ Chlor bedeutet.

7. Verbindung der Formel I nach Anspruch 1 bis 6, dadurch gekennzeichnet, dass die Gruppe -O-A in 4-Stellung am Phenylring steht.

8. Verbindung der Formel I nach Anspruch 1 bis 6, dadurch gekennzeichnet, dass die Gruppe -O-A in 5-Stellung am Phenylring steht.

9. Verbindung der Formel I nach Anspruch 1 bis 8, dadurch gekennzeichnet, dass

$R_1$ Wasserstoff, Fluor, Chlor oder Methyl;
$R_2$ Fluor, Chlor oder Trifluormethyl;
$R_3$ Methyl, Aethyl, n-Propyl, i-Propyl, $-CH_2CF_3$ oder $-CH_2CCl_3$;
$R_4$ Wasserstoff, Fluor, Chlor, Brom oder Methyl;
$R_5$ Wasserstoff, Fluor oder Chlor; und
$R_6$ und $R_7$ Wasserstoff
bedeuten.

10. Verbindung der Formel I nach Anspruch 9, dadurch gekennzeichnet, dass
$R_1$ Wasserstoff, Fluor oder Chlor;
$R_2$ Fluor;
$R_3$ Methyl, Aethyl oder n-Propyl; und
$R_4$ und $R_5$ unabhängig voneinander Wasserstoff, Fluor oder Chlor
bedeuten.

11. Verbindung der Formel I nach Anspruch 10, dadurch gekennzeichnet, dass
$R_3$ Methyl oder Aethyl und
$R_4$ und $R_5$ unabhängig voneinander Wasserstoff oder Chlor
bedeuten.

12. Verbindung der Formel I nach Anspruch 1 bis 8 und 11, dadurch gekennzeichnet, dass
$R_1$ Wasserstoff oder Brom und
$R_2$ Brom
bedeuten.

13. Verbindung der Formel I nach Anspruch 1 bis 8 und 12, dadurch gekennzeichnet, dass
$R_4$ Wasserstoff oder Methyl und
$R_5$ Methyl
bedeuten.

14. Verbindung der Formel I nach Anspruch 1 bis 8, 12 und 13, dadurch gekennzeichnet, dass

$R_3$ $C_1$-$C_3$-Halogenalkyl mit 1 bis 3 Halogenatomen, Allyl oder Propargyl

bedeutet.

15. Verbindung nach Anspruch 11 der Formel

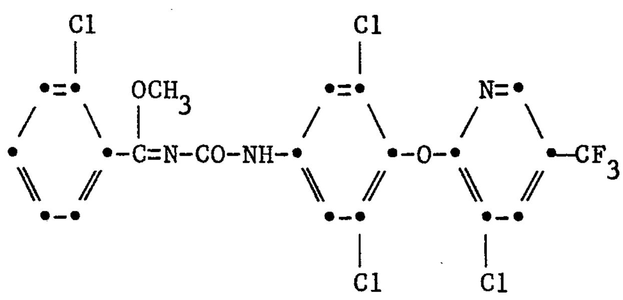

.

16. Verbindung nach Anspruch 13 der Formel

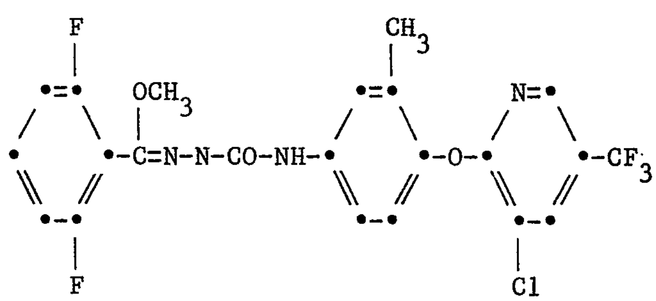

.

17. Verbindung nach Anspruch 11 der Formel

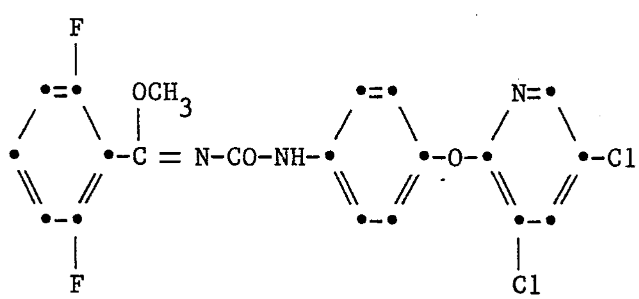

.

18. Verbindung nach Anspruch 12 der Formel

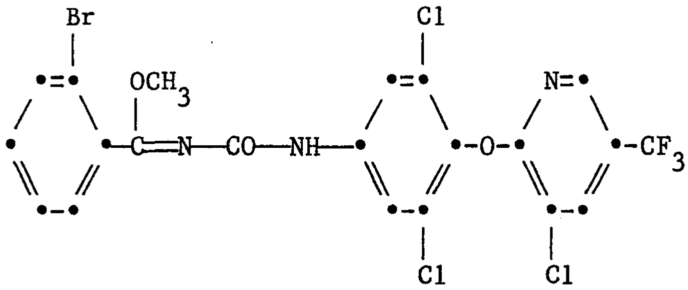

19. Verfahren zur Herstellung einer Verbindung der Formel I nach Anspruch 1 bis 18, dadurch gekennzeichnet, dass man

a) eine Verbindung der Formel II

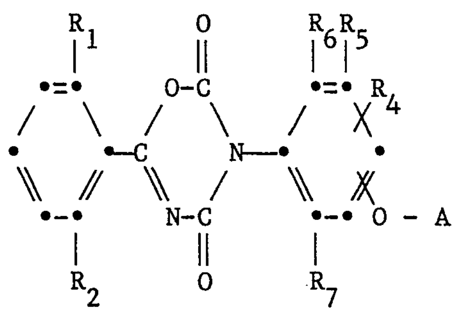

(II)

mit einer Verbindung der Formel III

$R_3$-OH (III)

oder

b) eine Verbindung der Formel IV

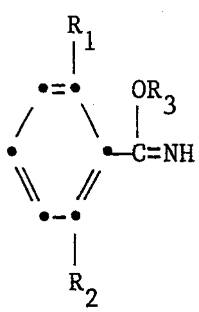

(IV)

mit einer Verbindung der Formel V

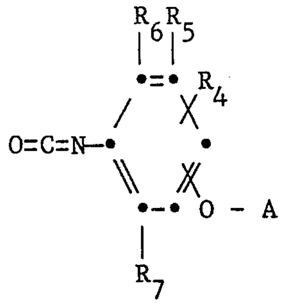

(V)

oder mit Phosgen und einer Verbindung der Formel VI

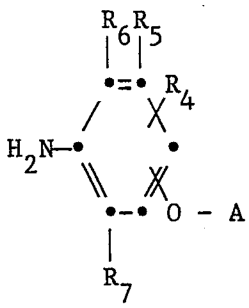

(VI)

umsetzt, wobei in den Formeln II bis VI die Reste $R_1$ bis $R_7$ und A die in den Ansprüchen 1 bis 14 angegebenen Bedeutungen haben.

20. Schädlingsbekämpfungsmittel, welches als aktive Komponente eine Verbindung gemäss den Ansprüchen 1 bis 18 zusammen mit geeigneten Trägern und/oder anderen Zuschlagstoffen enthält.

21. Verwendung einer Verbindung gemäss den Ansprüchen 1 bis 18 zur Bekämpfung von Insekten und Vertretern der Ordnung Akarina an Tieren und Pflanzen.

22. Verwendung gemäss Anspruch 21 zur Bekämpfung pflanzenschädigender Insekten.

23. Verwendung gemäss Anspruch 22 zur Bekämpfung larvaler Insektenstadien.

24. Verfahren zur Bekämpfung von Insekten und Vertretern der Ordnung Akarina, dadurch gekennzeichnet, dass man die Schädlinge bzw. deren verschiedene Entwicklungsstadien oder ihren Aufenthaltsort mit einer pestizid wirksamen Menge einer Verbindung der Formel I gemäss den Ansprüchen 1 bis 18 oder mit einem Mittel enthaltend neben Zusatz- und Trägerstoffen eine pestizid wirksame Menge dieser Verbindung, in Kontakt bringt oder behandelt.

FO 7.5/EIC/am*

0138772

Europäisches Patentamt

# EUROPÄISCHER RECHERCHENBERICHT

Nummer der Anmeldung

EP 84 81 0501

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-2 818 830 (ISHIHARA)<br>* Seiten 1-9 *<br>--- | 1,20 | C 07 D 213/00 |
| Y | US-A-4 431 667 (UPJOHN)<br>* Ansprüche 1-7 *<br>--- | 1,20 | |
| Y | GB-A-2 092 587 (UPJOHN)<br>* Seiten 1-4 *<br>--- | 1,20 | |
| Y,D | EP-A-0 005 944 (I.C.I.)<br>* Seiten 1-3 *<br>--- | 1,20 | |
| P,X | EP-A-0 109 211 (I.C.I.)<br>* Seiten 12-13 *<br>----- | 1-24 | |
| | | | RECHERCHIERTE SACHGEBIETE (Int. Cl.4) |
| | | | C 07 D 213/00 |

Der vorliegende Recherchenbericht wurde für alle Patentansprüche erstellt.

| Recherchenort | Abschlußdatum der Recherche | Prüfer |
|---|---|---|
| DEN HAAG | 28-01-1985 | VERHULST W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument